## Europäisches Patentamt
## European Patent Office
## Office européen des brevets

(19)

(11) Veröffentlichungsnummer: **0 019 767**
**B1**

(12)

# EUROPÄISCHE PATENTSCHRIFT

(45) Veröffentlichungstag der Patentschrift:
**01.08.84**

(51) Int. Cl.³: **C 01 C 1/24, C 07 C 131/00**

(21) Anmeldenummer: **80102512.3**

(22) Anmeldetag: **08.05.80**

(54) **Verfahren zur Behandlung von Cyclohexanon und Cyclohexanonoxim enthaltenden Ammoniumhydrogensulfatlösungen.**

(30) Priorität: **28.05.79 DE 2921649**

(43) Veröffentlichungstag der Anmeldung:
**10.12.80 Patentblatt 80/25**

(45) Bekanntmachung des Hinweises auf die Patenterteilung:
**01.08.84 Patentblatt 84/31**

(84) Benannte Vertragsstaaten:
**BE CH DE FR GB IT LI NL**

(56) Entgegenhaltungen:
**DE - A - 2 106 384**
**GB - A - 1 528 513**
**US - A - 3 940 442**

(73) Patentinhaber: **BASF Aktiengesellschaft,
Carl-Bosch-Strasse 38, D-6700 Ludwigshafen (DE)**

(72) Erfinder: **Fuchs, Hugo, Dr., Egellstrasse 28,
D-6700 Ludwigshafen (DE)**
Erfinder: **Hub, Dieter, Weinbrennerstrasse 20,
D-6830 Schwetzingen (DE)**

## Beschreibung

Gegenstand der Erfindung ist ein Verfahren zur Behandlung von Cyclohexanon und Cyclohexanonoxim enthaltenden wässrigen Ammoniumhydrogensulfatlösungen, die bei der Umsetzung von Cyclohexanon mit Hydroxylammoniumammoniumsulfat nach Abtrennen von Cyclohexanonoxim anfallen, bei dem man Cyclohexanonoxim bei erhöhter Temperatur zersetzt und Cyclohexanon azeotrop abdestilliert.

Bei der Herstellung von Cyclohexanonoxim durch Umsetzen von Hydroxylammoniumammoniumsulfatlösungen mit Cyclohexanon, die beispielsweise in der GB-PS 1 528 513 beschrieben wird, ist man bestrebt, nach Abtrennung des Cyclohexanonoxims Ammoniumhydrogensulfatlösungen zu erhalten, die wieder zur Herstellung von Hydroxylammoniumammoniumsulfat geeignet sind. Hierfür ist es notwendig, den Gehalt an organischem Kohlenstoff in der Ammoniumhydrogensulfatlösung sehr niedrig zu halten, da dies zur Schädigung des Katalysators bei der Hydroxylaminsynthese führt. Entsprechend der GB-PS 1 528 513 wird aus der nach der Oxymierung erhaltenen Ammoniumhydrogensulfatlösung Cyclohexanonoxim durch Erhitzen zersetzt und die gesamte Menge Cyclohexanon azeotrop abdestilliert. Solche Lösungen enthalten jedoch noch immer 0,02 Gew.-% organischen Kohlenstoff. Deshalb ist es erforderlich, solche Lösungen z.B. durch Behandlung mit Aktivkohle zu reinigen. Eine solche Arbeitsweise ist technisch aufwendig und deshalb verbesserungsbedürftig.

Es war deshalb die technische Aufgabe gestellt, bei der Cyclohexanonoximherstellung anfallende wässrige Ammoniumhydrogensulfatlösungen so zu behandeln, dass der Gehalt an organischem Kohlenstoff unter 100 ppm beträgt und die so erhaltenen Lösungen ohne Schädigung des Katalysators bei der Hydroxylaminsynthese verwendet werden können.

Diese Aufgabe wird gelöst in einem Verfahren zur Abtrennung von Restanteilen an Cyclohexanon und Cyclohexanonoxim aus wässrigen, im Kreislauf geführten Ammoniumhydrogensulfatlösungen, die bei der Umsetzung von Cyclohexanon mit Hydroxylammoniumammoniumsulfat im Rahmen der Hydroxylaminsynthese nach Abtrennen des erzeugten Cyclohexanonoxims anfallen, wobei der Restanteil an Cyclohexanonoxim bei erhöhter Temperatur zersetzt und das vorhandene sowie das bei der Zersetzung des Rest-Cyclohexanonoxims gebildete Cyclohexan azeotrop abdestilliert wird, dadurch gekennzeichnet, dass man die Ammoniumhydrogensulfatlösung in einer ersten Stufe in den oberen einer mit je einem oberen und unteren Verdampfer ausgerüsteten Kolonne einführt, in diesem oberen Verdampfer das Cyclohexanon azeotrop abdestilliert und in der nachfolgenden Kolonne in einer zweiten Stufe das in der Ammoniumhydrogensulfatlösung enthaltene Cyclohexanonoxim bei einer Temperatur von 90–115 °C zersetzt, wobei das bei der Zersetzung freiwerdende Cyclohexanon in dem Masse wie es entsteht nach oben azeotrop abdestilliert und am unteren Ende der Kolonne die behandelte Ammoniumhydrogensulfatlösung entnommen und ohne weitere Vorbehandlung in die Hydroxylaminsynthese zurückgeführt wird.

Das neue Verfahren hat den Vorteil, dass es ohne grossen technischen Aufwand gelingt, den Kohlenstoffgehalt der Ammoniumhydrogensulfatlösung erheblich abzusenken, so dass solche Lösungen ohne Schädigung des Katalysators bei der Hydroxylaminsynthese verwendbar sind. Das neue Verfahren ist insofern auch bemerkenswert, als es gelingt, das als schädliches Nebenprodukt in der Ammoniumhydrogensulfatlösung enthaltene Cyclohexenylcyclohexanon abzutrennen.

Entsprechend dem in der US-PS 3 940 442 beschriebenen Verfahren soll eine wässrige Lösung, die Phosphorsäure oder Ammoniumbisulfat enthält, zur Reduzierung von Verunreinigungen, die aus der Oximierung stammen, eine ausreichende Zeit auf 50 bis 106 °C erhitzt werden, um für eine Hydroxylaminsynthese, ausgehend von Salpetersäure unter Mitverwendung eines Paladiumkatalysators, verwendbar zu sein. Die zu behandelnde Lösung enthält zwangsweise oxidierend wirkende Nitrationen, und es soll zusätzlich oxidierend wirkende Salpetersäure zugestzt werden.

Ferner ist der US-PS zu entnehmen, dass die dort beschriebene Behandlung in Gegenwart von Ammoniumsulfat Schwierigkeiten bereitet und falls zusätzlich das zu behandelnde Gemisch stark sauer ist, nicht abtrennbare organische Verbindungen entstehen, die zur Schädigung des Katalysators in der Hydroxylaminsynthese führen.

Bei dem erfindungsgemäss zu behandelnden Gemisch handelt es sich um eine Lösung, die Ammoniumsulfat enthält und aufgrund des Gehaltes von Ammoniumhydrogensulfat von sich aus stark sauer ist. Zudem sind oxidierend wirkende Nitrationen von der Herstellung her nicht enthalten. Es war somit davon auszugehen, dass die in der Entgegenhaltung beschriebene Reinigungsmethode für die in Frage kommende Lösung nicht erfolgversprechend ist. Führt man die Reinigung der Ammoniumbisulfat enthaltenden Ausgangslösung in einer Strippkolonne durch, wie in der US-PS Spalte 5, Zeilen 60 bis 66, und in Beispiel B 1 empfohlen, so führt dies ausweislich des Vergleichsbeispiels nicht zum gewünschten Erfolg. Es war deshalb allenfalls der Schluss zu ziehen, dass oxidierend wirkende Mittel mitverwendet werden müssen.

Die erfindungsgemäss zu behandelnden Cyclohexanon und Cyclohexanonoxim enthaltenden Ammoniumhydrogensulfatlösungen fallen bei der Umsetzung von Cyclohexanon mit Hydroxylammoniumammoniumsulfat nach Abtrennen von Cyclohexanonoxim an. Beispielsweise wird eine wässrige Lösung von Hydroxylammoniumammoniumsulfat bei Temperaturen oberhalb des Schmelzpunktes von Cyclohexanonoxim im Gegenstrom mit Cyclohexanon umgesetzt, wobei man einen pH-Wert von nicht grösser als 1 einhält, bis der Gleichgewichtszustand erreicht ist. Das Cyclohexanonoxim wird flüssig abgezogen und die erhaltene wässrige Lösung zweckmässig nochmals im Gegenstrom mit frischem Cyclohexanon extrahiert, um gelöstes Cyclohexanonoxim abzutrennen. Eine typische Lösung enthält beispielsweise 15 bis 35 Gew.-% Ammoniumhydrogensulfat, 1 bis 5 Gew.-% Ammoniumsulfat, 0 bis 1 Gew.-% Hydroxylammoniumsulfat ger. als Hydroxylamin sowie 0,1 bis 5 Gew.-%, insbesondere 0,5 bis 3,0 Gew.-% Cyclohexanon und Cyclohexanonoxim sowie weitere Verunreinigungen organischer Natur wie Cyclohexenylcyclohexanon. Eine solche Lösung hat in der Regel einen pH-Wert von nicht grösser als 1, vorzugsweise von nicht grösser als 0,5. Ein geeignetes Verfahren, wie man solche Lösungen erhält, wird beispielsweise in der GB-PS 1 528 513 beschrieben.

Aus der zu behandelnden Ammoniumhydrogensulfatlösung wird zunächst in einer ersten Stufe das darin enthaltene Cyclohexanon azeotrop abdestilliert. Hierbei hält man vorteilhaft Temperaturen von 93 bis 105 °C, insbesondere 95 bis 100 °C ein. Die anzuwendenden Temperaturen richten sich im wesentlichen nach dem Siedepunkt des Azeotrops Wasser Cyclohexanon und dem jeweils herrschenden Druck. In der Regel verwendet man Atmosphärendruck, es ist jedoch auch möglich, schwachen Unterdruck oder mässigen Überdruck, z.B. bis zu 1,5 bar, anzuwenden.

In einer zweiten Stufe wird das noch in der Ammoniumhydrogensulfatlösung enthaltene Cyclohexanonoxim bei einer Temperatur von 90 bis 115 °C, insbesondere 95 bis 110 °C, zersetzt und das freiwerdende Cyclohexanon in dem Masse wie es entsteht azeotrop abdestilliert. Erfindungsgemäss wird demnach die Entfernung des ursprünglich in der Ammoniumhydrogensulfat enthaltenen Menge an Cyclohexanon räumlich getrennt von der Zersetzung des Cyclohexanonoxims vorgenommen.

Man verfährt so, dass man die zu behandelnde Ammoniumhydrogensulfatlösung in den oberen Teil einer Kolonne, der mit einem Verdampfer ausgerüstet ist, zuführt. Hierbei wird Cyclohexanon azeotrop bei den vorgenannten Temperaturen abdestilliert. Im Verlauf der Kolonne nach unten wird dann das in der Ammoniumhydrogensulfatlösung noch enthaltene Cyclohexanonoxim bei den angegebenen Temperaturen zersetzt. Vorzugsweise weist die Kolonne 2 bis 10 theoretische Böden auf. Es hat sich bewährt, wenn man für die Zersetzung des Cyclohexanonoxims eine Verweilzeit von 1 bis 20 Min. einhält. Das freiwerdende Cyclohexanon destilliert dann azeotrop mit Wasser über den Kopf der Kolonne ab. Um dies zu gewährleisten, hält man im Sumpf der Kolonne zweckmässig eine Temperatur von 100 bis 115 °C ein. Am unteren Ende der Kolonne wird wässrige Ammoniumhydrogensulfatlösung entnommen, die noch Ammoniumsulfat und Hydroxylammoniumsulfat entsprechend der verwendeten Ausgangslösung enhält und einen Gehalt an organischem Kohlenstoff von weniger als 100 ppm aufweist.

Die so erhaltene Lösung ist ohne weitere Vorbehandlung zur Herstellung von Hydroxylammoniumammoniumsulfat durch Reduktion von Stickstoffmonoxid mit Wasserstoff in Gegenwart von Platin-Trägerkatalysatoren geeignet.

Das Verfahren nach der Erfindung sei an folgenden Beispielen veranschaulicht.

Beispiel

Auf eine pulsierte, mit Raschigringen gefüllte Glaskolonne werden am oberen Ende stündlich 20,060 kg einer Hydroxylammoniumammoniumsulfatlösung folgender Zusammensetzung aufgegeben:
14,685 kg $H_2O$
5,167 kg $(NH_3OH-NH_4)SO_4$
0,104 kg $NH_4HSO_4$
0,104 kg $(NH_4)_2SO_4$

Am unteren Ende werden stündlich 3,908 kg Cyclohexanon mit einem Anteil an frischem Cyclohexanon von 3,468 kg eingebracht. Die Reaktionstemperatur beträgt 90 °C im oberen Teil und 70 °C im unteren Teil der Kolonne.

Aus dem oberen Teil der Kolonne können stündlich 4,245 kg Cyclohexanonoxim mit einem Anonanteil von ca. 2% und einem Wassergehalt von 6,1% abgezogen werden.

Die am unteren Ende der Pulsationskolonne anfallende Wasserphase von 19,723 kg hat einen pH-Wert von = 0,5 bei 25 °C und ist folgendermassen zusammengesetzt:
15,053 kg Wasser
0,405 kg Cyclohexanon
0,041 kg Cyclohexanonoxim
4,114 kg $NH_4HSO_4$
0,104 kg $(NH_4)_2SO_4$
0,006 kg $(NH_3OH-NH_4)SO_4$

Eine Glockenbodenkolonne, welche unten und oben mit je einem Verdampfer versehen ist, wird stündlich mit 19,723 kg der beschriebenen Lösung beaufschlagt. Die Lösung wird dem oberen Verdampfer zugeführt. Die Kolonne hat einen Durchmesser von 80 mm und enhält 10 Glockenböden. Die Sumpftemperatur beträgt 104 °C und wird bei Normaldruck betrieben. Am Kopf der Kolonne werden 100 °C gemessen. Über Kopf der Kolonne werden stündlich 11 kg eines Gemisches aus Cyclohexanon und Wasser abdestilliert und kondensiert. Das Kondensat trennt sich in eine Cyclohexanon- und eine Wasserphase. Die Anonphase von 0,440 kg wird dem Anonzulauf der Reaktionskolonne zugeführt.

Auf diese Weise wird eine schnelle Trennung des im Zulauf befindlichen Anons von der wässrigen Ammoniumhydrogensulfatlösung erreicht. Der obere Verdampfer kann ohne Sumpf betrieben werden. Im weiteren Verlauf der Kolonne hydrolysiert das Cyclohexanonoxim zu Hydroxylamin und Cyclohexanon. Letzteres wird ebenfalls durch Destillation aus der Ammoniumhydrogensulfatlösung entfernt.

Die am unteren Ende der Kolonne anfallende wässrige Ammoniumhydrogensulfatlösung hat folgende Zusammensetzung:
15,046 kg $H_2O$
4,072 kg $NH_4HSO_4$
0,060 kg $(NH_3OH-NH_4)SO_4$

0,104 kg $(NH_4)_2SO_4$
Kohlenstoffgehalt 60 ppm

Diese Lösung wird nun in Gegenwart eines Platinkatalysators bie 40 °C und unter intensivem Rühren mit einem Gemisch aus Wasserstoff und Stickstoffmonoxid im Volumenverhältnis 2:1 behandelt. Die erhaltene Hydroxylammoniumammoniumsulfatlösung kann wieder in den Oximierungskreislauf zurückgeführt werden. Kleine Mengen von sich bildendem Ammoniumsulfat werden durch Ausschleusen der entsprechenden Menge Lösung vor der Hydroxylaminsynthese entfernt oder können nach bekannten Methoden, z.B. durch Behandlung mit Stickoxiden, zersetzt werden.

Vergleichsbeispiel

Man führt wie in Beispiel 1 beschrieben der Destillationskolonne, welche am oberen Ende keinen separaten Verdampfer enthält, stündlich 19,723 kg der am unteren Teil der Reaktionskolonne anfallenden Wasserphase der gleichen Zusammensetzung zu. Über Kopf der Kolonne werden die gleichen Mengen Anon und Wasser wie in Beispiel 1 abdestilliert und kondensiert. Die Anonphase wird wieder dem Frischanonzulauf und die Wasserphase der Kolonne zugeführt.

Die am unteren Teil der Kolonne anfallende wässrige Ammoniumhydrogensulfatlösung hat folgende Zusammensetzung:
15,046 kg $H_2O$
 4,072 kg $NH_4HSO_4$
0,060 kg $(NH_3OH–NH_4)SO_4$
0,104 kg $(NH_4)_2SO_4$
Kohlenstoffgehalt 200 ppm

## Patentanspruch

Verfahren zur Abtrennung von Restanteilen an Cyclohexanon und Cyclohexanonoxim aus wässrigen, im Kreislauf geführten Ammoniumhydrogensulfatlösungen, die bei der Umsetzung von Cyclohexanon mit Hydroxylammoniumammoniumsulfat im Rahmen der Hydroxylaminsynthese nach Abtrennen des erzeugten Cyclohexanonoxims anfallen, wobei der Restanteil an Cyclohexanonoxim bei erhöhter Temperatur zersetzt und das vorhandene sowie das bei der Zersetzung des Rest-Cyclohexanonoxims gebildete Cyclohexanon azeotrop abdestilliert wird, dadurch gekennzeichnet, dass man die Ammoniumhydrogensulfatlösung in einer ersten Stufe in den oberen einer mit je einem oberen und einem unteren Verdampfer ausgerüsteten Kolonne einführt, in diesem oberen Verdampfer das Cyclohexanon azeotrop abdestilliert und in der nachfolgenden Kolonne in einer zweiten Stufe das in der Ammoniumhydrogensulfatlösung enthaltene Cyclohexanonoxim bei einer Temperatur von 90 bis 115 °C zersetzt, wobei das bei der Zersetzung freiwerdende Cyclohexanon in dem Masse wie es entsteht nach oben azeotrop abdestilliert und am unteren Ende der Kolonne die behandelte Ammoniumhydrogensulfatlösung entnommen und ohne weitere Vorbehandlung in die Hydroxylaminsynthese zurückgeführt wird.

## Claim

A process for the separation of residual amounts of cyclohexanone and cyclohexanone-oxime from äqueous recycled ammonium bisulfate solutions which are formed in the reaction of cyclohexanone with hydroxylammonium ammonium sulfate, in the synthesis of hydroxylamine, after removal of the cyclohexanone-oxime produced, the residual amount of cyclohexanone-oxime being decomposed at elevated temperature, and the cyclohexanone which is present and that formed in the decomposition of the residual cyclohexanone-oxime being distilled off azeotropically, wherein, in a first stage, the ammonium bisulfate solution is fed into the upper vaporizer of a column equipped with an upper and a lower vaporizer, the cyclohexanone is distilled off azeotropically in this upper vaporizer and, in a second stage, the cyclohexanone-oxime contained in the ammonium bisulfate solution is decomposed in the subsequent portion of the column at a temperature of from 90 to 115 °C, the cyclohexanone liberated in the decomposition being distilled off azeotropically, in an upward direction, at the rate at which it is formed, and the treated ammonium bisulfate solution being removed at the lower end of the column and recycled, without further pretreatment, to the hydroxylamine synthesis.

## Revendication

Procédé pour la séparation de fractions résiduelles de cyclohexanone et d'oxime de la cyclohexanone de solutions aqueuses d'hydrogénosulfate d'ammonium telles qu'obtenues dans la synthèse de l'hydroxyl-amine par réaction de la cyclohexanone et du sulfate d'ammonium et d'hydroxyl-ammonium après la séparation de l'oxime de la cyclohexanone formé, circulant en circuit fermé, la fraction résiduelle d'oxime de la cyclohexanone étant décomposée à température accrue et la cyclohexanone présente et (ou) formée lors de la décomposition de l'oxime de la cyclohexanone résiduel étant séparée par distillation azéotrope, caractérisé en ce que, dans un premier stade, on introduit la solution d'hydrogéno-sulfate d'ammonium dans l'évaporateur supérieur d'une colonne comprenant un évaporateur supérieur et un évaporateur inférieur, on y sépare la cyclohexanone par distillation azéotrope et, dans un deuxième stade, on décompose dans la colonne subséquente l'oxime de la cyclohexanone contenu dans la solution d'hydrogéno-sulfate d'ammonium à une température de 90 à 115 °C, la cyclohexanone libérée par la décomposition se séparant vers le haut, au fur et à mesure de sa formation, par distillation azéotrope et la solution d'hydrogéno-sulfate d'ammonium traitée étant soutirée à l'extrémité inférieure de la colonne et recyclée sans autre traitement dans la synthèse de l'hydroxyl-amine.